Europäisches Patentamt

European Patent Office

Office européen des brevets

(19)

(11) Publication number: **0 393 707 B1**

# EUROPEAN PATENT SPECIFICATION

(45) Date of publication of patent specification: **26.10.94**   (51) Int. Cl.5: **A61K 9/127**, A61K 37/02

(21) Application number: **90107549.9**

(22) Date of filing: **20.04.90**

The file contains technical information submitted after the application was filed and not included in this specification

(54) **Bioactive compounds associated with liposomes and their use in pharmaceutical preparations.**

(30) Priority: **21.04.89 US 341390**
**06.04.90 US 505584**

(43) Date of publication of application:
**24.10.90 Bulletin 90/43**

(45) Publication of the grant of the patent:
**26.10.94 Bulletin 94/43**

(84) Designated Contracting States:
**CH DE DK ES FR GB IT LI NL SE**

(56) References cited:
**EP-A- 0 256 989**
**EP-A- 0 356 340**
**WO-A-89/05149**
**DE-A- 3 301 951**

**CHEMICAL ABSTRACTS vol. 100, no. 17, 23 April1984, page 75, abstract no. 132701x, Columbus, Ohio, US; B. GYSIN et al.:"Hydrophobic and Electrostatic Interactions between Adrenocorticotropin-(1-24)-tetrapeptide and Lipid Vesicles. Amphiphilic Primary Structures" &Biochemistry 1984, vol. 23, no. 8, pages 1811-1818**

**JOURNAL OF PHARMACEUTICAL SCIENCES**

**vol. 79, no. 3, March 1989,pages 232-235, Washington, US; E. SADA et al.: "Effects of Surface Charges andCholesterol Content on Amino Acid Permeabilities of Small Unilamellar Vesicles"**

(73) Proprietor: **OTSUKA PHARMACEUTICAL CO., LTD.**
**9, Kandatsukasa-cho 2-chome**
**Chiyoda-ku Tokyo 101 (JP)**

(72) Inventor: **Strassmann, Gideon**
**2800 Woodley Road,**
**N.W. No. 212**
**Washington D.C. 20008 (US)**

(74) Representative: **Hansen, Bernd, Dr.**
**Dipl.-Chem. et al**
**Hoffmann, Eitle & Partner**
**Patent- und Rechtsanwälte,**
**Postfach 81 04 20**
**D-81904 München (DE)**

EP 0 393 707 B1

**Description**

FIELD OF THE INVENTION

The present invention relates to a method of treating a patient with bioactive compounds such as transforming growth factor, type $\beta$, hereinafter "TGF-$\beta$" and colony-stimulating factors, such as macrophage colony-stimulating factor, hereinafter "M-CSF." In addition, the present invention relates to compositions of bioactive compounds associated with liposomes such as TGF-$\beta$ associated with liposomes and M-CSF associated with liposomes. More particularly, the invention relates to pharmaceutical compounds comprising a bioactive compound associated with ionically-charged liposomes in conjunction with a pharmaceutically-acceptable carrier for administration to patients. Such pharmaceutical compounds may be TGF-$\beta$ associated with liposomes or M-CSF associated with liposomes.

BACKGROUND OF THE INVENTION

Bioactive compounds such as TGF-$\beta$ and M-CSF may be administered for the treatment of a variety of diseases. The administration of such bioactive compounds, however, may be hampered by limited bioavailability. Once a biological agent is absorbed or injected into the blood stream, the rate, extent, and pattern of the initial distribution are determined, inter alia, by the physicochemical characteristics of the drug. Compounds that exhibit a charge at physiologic pH may reach the target area in only diminished quantities. For example, the positive charge of TGF-$\beta$ may cause the peptide to adhere to cellular membranes, such as vascular membranes, or to plasma proteins, such as albumin, or other components of blood such that the half-life of the agent is decreased and biological activity is reduced. Similarly, the negative charge of M-CSF may hamper the ability of that agent to reach the target site.

Any reduction in bioavailability is exacerbated by administration through the blood system because compounds in the blood stream are vulnerable to the body's clearing mechanisms. Recently, liposomes have been used to protect various biologically active agents from such host clearing mechanisms by entrapment of the biological agent within the liposome.

Entrapment of biologically active agents within liposomes, however, requires a relatively large amount of agent. This is problematic for peptides, like TGF-$\beta$, that are produced at great expense and in very small quantities. For example, an improved method for the production of TGF-$\beta$, described as presenting a five-fold increase in recovery over previous isolation techniques, yields only 2.5 $\mu$g per unit of human platelets. Cone et al. "An Improved Method of Purification of Transforming Growth Factor, Type Beta from Platelets," Analytical Biochemistry 168: 71-74 (1988). Because relatively large quantities of biologically active agent are dispersed in an aqueous layer in the preparation of a liposome-entrapped biological agent, this method is not suitable for use with peptides that are not readily available in large quantities, such as TGF-$\beta$.

In addition, liposome entrapment of biological agents may be inferior to liposome surface association with biological agents, such as TGF-$\beta$ and M-CSF, that act by first attaching to receptors on the membranes of cells because agents entrapped within the aqueous layer of a liposome will not have access to cellular receptors. Recent studies have shown that TGF-$\beta$ and M-CSF do attach to cellular receptors found on a wide variety of different cell types. Wakefield, L.M. et. al., "Distribution and Modulation of the Cellular Receptor for Transforming Growth Factor-Beta," J. Cell. Bio. 105: 965-975 (1987). Stanley, E.R. et al., "Regulation of macrophage production by colony-stimulating factor", in Mononuclear Phagocytes - Functional Aspects, Part 1, ed. R. van Furth (1980); Byrene, P.V., et al., "This Distribution of Cells Having Receptors for a Colony-Stimulating Factor (CSF-1) in Murine Tissues," J. Cell. Biol. 91:848 (1981). Thus, entrapment of TGF-$\beta$ and M-CSF in liposomes may diminish biological activity of the bioactive agents.

Accordingly, there is a need in the art for a method of administering bioactive agents such as TGF-$\beta$ and M-CSF to patients that will maximize the quantity of biological agent that reaches the target site, protect the bioactive agents from the body's clearing mechanisms, and maintain the biological activity of the bioactive agents, and that is suitable in use with a peptide that is obtainable in small amounts. There is also a need in the art for compounds and compositions for use in the method.

SUMMARY OF THE INVENTION

The present invention overcomes the problems and disadvantages of the prior art by providing a method of treating a patient with bioactive agents, comprising providing aggregates of bioactive agents in association with liposomes, wherein the aggregates comprise liposomes having outer surfaces and bioactive agents, wherein the bioactive agent is associated with the outer surfaces of the liposomes such that biological activity is not substantially diminished when the aggregates are administered in a pharmaceutically effective amount to the patient, and administering the aggregates to the patient. The present invention also provides a method of treating patients with TGF-$\beta$, comprising providing aggregates of TGF-$\beta$ in association with liposomes, wherein the aggregates comprise liposomes having outer surfaces, and TGF-$\beta$, wherein the TGF-$\beta$ is associated with the outer surfaces of the liposomes such that biological activity is not substantially diminished when the aggregates are administered in a pharmaceutically effective amount to the patient, and administering the aggregates to the patient. The invention also provides a preferred embodiment of the method of treating patients with TGF-$\beta$ wherein negatively-charged liposomes are used to form the aggregates of TGF-$\beta$ in association with liposomes. In addition, the present invention provides a composition of matter, comprising aggregates of TGF-$\beta$ in association with liposomes. The invention also provides a pharmaceutical composition comprising pharmaceutically effective amounts of the aggregates of TGF-$\beta$ in association with liposomes in conjunction with pharmaceutically-acceptable carriers.

The present invention also provides a method of treating patients with M-CSF, comprising providing aggregates of M-CSF in association with liposomes, wherein the aggregates comprise liposomes having outer surfaces, and M-CSF, wherein the M-CSF is associated with the outer surfaces of the liposomes such that biological activity is not substantially diminished when the aggregates are administered in a pharmaceutically effective amount to the patient, and administering the aggregates to the patient. The invention also provides a preferred embodiment of the method of treating patients with M-CSF wherein positively-charged liposomes are used to form the aggregates of M-CSF in association with liposomes. In addition, the present invention provides a composition of matter, comprising aggregates of M-CSF in association with liposomes. The invention also provides a pharmaceutical composition comprising pharmaceutically effective amounts of the aggregates of M-CSF in association with liposomes in conjunction with pharmaceutically-acceptable carriers.

Additional objects and advantages of the invention will be set forth in part in the description which follows, and in part may be learned from the description or by practice of the invention. The objects and advantages of the invention will be realized and attained by means of the instrumentalities and combinations particularly pointed out in the appended claims.

BRIEF DESCRIPTION OF THE DRAWINGS

The accompanying drawings, which are incorporated in and constitute a part of this specification, illustrate several exemplary embodiments of the invention and, together with the description, serve to explain the principles of the invention.

Fig. 1 is a graph which depicts the effect of varying lipid/protein ratios on the association of TGF-$\beta$ with negatively-charged liposomes.

Fig. 2 is a graph which indicates the extent of TGF-$\beta$ association with neutral liposomes.

Fig. 3 is a graph which indicates the extent of TGF-$\beta$ association with negatively-charged liposomes.

Fig. 4 is a graph which depicts the effects of TGF-$\beta$ associated liposomes on delayed type hypersensitivity responses to Listeria.

Fig. 5 is a graph which depicts the effects of TGF-$\beta$ associated liposomes on delayed type hypersensitivity responses to Listeria.

DESCRIPTION OF THE PREFERRED EMBODIMENTS

Reference will now be made in detail to the presently preferred embodiments of the invention, examples of which are illustrated in the accompanying drawings.

As used herein, bioactive agents are peptides, polypeptides, proteins, and glycoproteins that cause a cellular response when administered to patients. The cellular responses observed with bioactive agents range from proliferative effects, nonproliferative effects, differentiation, and other effects such as the suppression of immunoglobulin secretion and adrenal steroidogenesis. The bioactive agents particularly preferred are TGF-$\beta$ and M-CSF.

As used herein, TGF-$\beta$ is a hormonally active polypeptide known as transforming growth factor, type beta. Much progress has been made in identifying and structurally characterizing TGF-$\beta$ since its discovery only a few years ago. TGF-$\beta$ consists of two identical polypeptide chains (Mr 12,500), linked by disulfide bonds. The activity of the native molecule is destroyed upon reduction of the disulfide groups.

TGF-$\beta$ is a ubiquitous molecule that is present in many normal tissues, as well as in transformed cells. Blood platelets, in particular, store relatively large amounts of TGF-$\beta$ (about 1500 molecules of TGF-$\beta$ per cell), which supports the view that TGF-$\beta$ has a role in wound healing.

TGF-$\beta$, however, has been found to be a multifunctional growth factor. For example, it has proliferative effects on fibroblasts and osteoblasts and anti-proliferative effects on fibroblasts, epithelial cells, T-lymphocytes, and osteoblasts. In addition, TGF-$\beta$ has effects that are unrelated to proliferation: it has been shown to suppress immunoglobulin secretion and adrenal steroidogenesis. Sporn and Roberts, "Peptide Growth Factors are Multifunctional," Nature 332: 217-219 (March 17, 1988). It has been suggested that the nature of growth factor action may depend on the presence of other substances, i.e., TGF-$\beta$ stimulates fibroblasts in the presence of platelet-derived growth factor but inhibits their growth in the presence of epidermal growth factor.

In addition, TGF-$\beta$ is believed to be a potent regulator of the expression of the processes of adipogenesis, myogenesis, osteogenesis, and epithelial differentiation. Scientists have proposed that the biochemical basis of these multiple cellular actions is due, at least in part, to the ability of TGF-$\beta$ to alter the architecture of extracellular matrices. See "The Transforming Growth Factors" by Joan Massague and "Negative Regulators of Cell Growth" by John L. Wang and Yen-Ming Hsu, in Oncogenes and Growth Factors, ed. Ralph A. Bradshaw and Steve Prentis (1987).

One function of TGF-$\beta$ that is of particular interest in this invention is its ability to manipulate the immune system. Recent studies show that TGF-$\beta$ is at least $10^4$ times more potent on a molar basis than the peptide cyclosporin A as a suppressor of T-lymphocytes. Sporn and Roberts, supra. This inhibitory effect makes it potentially useful in the treatment of conditions such as rheumatoid arthritis, systemic lupus erythematosis, autoimmune diabetes, autoimmune throiditis, and multiple sclerosis and other autoimmune diseases. In addition, TGF-$\beta$ may facilitate allograft transplantation and inhibit certain tumor cells.

Because TGF-$\beta$ has a pI of 9.5, Seyedin et al., "Cartilage-inducing Factor-B is a Unique Protein Structurally and Functionally related to Transforming Growth Factor-$\beta$," J. of Biol. Chem. 262 (5): 1946-49 (1987), at physiologic pH, it has been shown that TGF-$\beta$ has a net positive charge. Net positive charge, as used herein, refers to the positive ionization state of the polypeptide. Because polypeptides are composed of amino acids that have a certain charge at a particular pH, the ionization states of the individual components yield a net positive or negative charge for the polypeptide. The amino acid composition of TGF-$\beta$ is such that it bears a net positive charge.

As used herein, the term "M-CSF" relates to a particular type of colony-stimulating factor. Colony-stimulating factors are glycoproteins that regulate the production of hematopoietic cells. At least four distinct colony-stimulating factors control proliferation and differentiation of granulocytes and/or macrophages from hematopoietic precursors.

One particularly preferred agent is the macrophage colony-stimulating factor (M-CSF, also known as CSF-1) that can selectively stimulate the survival, proliferation, and differentiation of mononuclear phagocyte lineage cells. Studies have also shown that M-CSF also stimulates effector functions of mature monocytes such as antifungal activity and lymphokine-induced tumoricidal activity. Stanley, E.R. and Gulbert, L.T., "Method for the Purification, Assay, Characterization and Target Cell Binding of Colony-Stimulating Factor [CSF-1]", J. Immunologic Methods 42: 253-284 (1981); Karbassi, A., et al., "Enhanced Killing of Candida Albicans by Murine Macrophages Treated with Macrophage Colony-Stimulating Factor: Evidence for Augmented Expression of Mannose Receptors", J. Immunology 139: 417-421 (1987); Metcalf, D. "The Molecular Biology and Functions of the Granulocyte macrophage Colony-Stimulating Factors," Blood 67:257 (1986); and Ralph P. and Nakoinz, I., "Stimulation of Macrophage Tumoricidal Activity by Growth and Differentiation Factor CSF-1", Cellular Immunology 105: 270-279 (1987).

The instant application encompasses all forms of M-CSF, including whole intact M-CSF as well as M-CSF that has been truncated or otherwise altered by standard biochemical or recombinant techniques. See, U.S. Application Serial No. 07/304,692, filed February 1, 1989, entitled "Human Colony-Stimulating Factors," to Takahashi, M. et. al.

Studies have shown that, at physiological pH, human M-CSF exhibits a pI of 4.8 and a net negative charge. Similarly, a truncated form of human M-CSF has been shown to exhibit a PI of 4.5. Such recombinant techniques are provided in the copending U.S. Application Serial No. 07/304,692, filed February 1, 1989, entitled "Human Colony-Stimulating Factors," to Takahashi, M. et. al. Net negative charge, as used herein, refers to the negative ionization state of the agent. As discussed above with regard to net positive charge, the charge of an entity is based on the amino acids that comprise the entity. The amino acids that make up at least a part of a glycoprotein confers on the glycoprotein a particular charge.

As used herein, the term "aggregate" means a collection of units or particles into a body, mass, or other grouping. The present invention is not limited by the number of units that form the aggregate of the present invention.

The term "association," as used herein, means any kind of relationship between the parts of the association. The parts may be associated by proximity so that there is no actual contact between the parts.

Additionally, the parts may be associated by superficial contact, such as surface-to-surface contact. The association may result from even closer contact such that a member of the association is embedded, partially or fully, in another member of the association.

As used herein, the term "liposome" is to be given a broad meaning. Liposomes, also called lipid vesicles, are aqueous compartments enclosed by a lipid bilayer which range in shape from spherical to elongated.

Liposomes are formed by suspending a suitable lipid in an aqueous medium. This mixture is then shaken to yield a dispersion of liposomes that are quite uniform in size. Alternatively, liposomes can be made by rapidly mixing a solution of lipid in ethanol with water. This can be accomplished by injection of the lipid through a fine needle. The method chosen to prepare a liposome in part determines the form of liposome prepared. The present invention contemplates use of all forms of liposomes, including large unilamellar vesicles, small unilamellar vesicles, and multilamellar vesicles.

Multilamellar vesicles, also called oligolamellar vesicles, can be prepared in any conventional manner, including that employing a rotary vacuum evaporator. Sunamato, J. et al., "A Newly Developed Immunoliposome - an Egg Phosphatidyl Choline Liposome Coated with Pullulan bearing both a Cholesterol Moiety and a IgMs fragment," Biochem. Biophys. Acta 898: 323-330 (1987).

Large unilamellar vesicles can also be prepared in any conventional manner, such as by an extrusion procedure. Hope, M.J., et al. "Production of Large Unilamellar Vesicles by a Rapid Extrusion Procedure, Characterization of Size, Distributions, Trapped Volumes and Ability to Maintain a Membrane Potential", Biochem. Biophys. Acta 812: 55-65 (1985). Alternatively, small unilamellar vesicles can be prepared using conventional techniques such as sonication. A preferred technique for the preparation of small unilamellar vesicles is described below.

Generally, liposomes can be prepared from a variety of lipid material, including for example, phosphatidyl choline, ovolecithin, cholesterol, and sterylamino, among others. Liposomes prepared with lipid materials such as phosphatidyl choline are neutral in charge. In a preferred embodiment, however, the liposomes are prepared from lipids that will form negatively charged liposomes. Lipid materials such as, but not limited to, phosphatidyl serine, dicetyl phosphate and dimyristoyl phosphatidic acid, are examples of lipid materials that form the preferred negatively-charged liposomes. Although any charged lipid material is suitable, in a particularly preferred embodiment, phosphatidyl choline (PC) and phosphatidyl serine (PS) are combined to form negatively-charged liposomes. PC and PS may be combined in any ratio. In a particularly preferred embodiment, PC and PS are combined at a molar ratio of 1:1.

Alternatively, lipid materials such as stearylamine may be used to form positively charged liposomes.

The lipid components can be mixed with chloroform, and dried to a thin film under a stream of nitrogen. The dried lipidic material is then rehydrated. The lipidic material of the present invention is preferably rehydrated by the addition of phosphate buffered saline (pH 7.2, without Ca2 + or Mg2 + ) for 30 minutes at room temperature. The liposomes of the invention are formed by physically shaking the solution of rehydrated lipidic material to form small unilamellar vesicles. Although mechanical or ultrasonic shaking can be used, in a preferred embodiment, the small unilamellar vesicle type liposomes of the present invention are formed by sonication in a both sonicator for one hour and/or until no further optical clearance of the solution can be observed.

In one aspect of the present invention, the outer surface of liposome is associated with a bioactive agent such as TGF-$\beta$ or M-CSF. As described above, this association refers to any kind of relationship between the parts of the association. The outer surface of the liposome may be associated by proximity to TGF-$\beta$ or M-CSF so that no actual contact between the parts exists. Alternatively, the outer surface of the liposomes may be associated with TGF-$\beta$ or M-CSF by superficial contact so that the outer surface of the liposome is in contact with TGF-$\beta$ or M-CSF. In another embodiment, the outer surface of liposomes may be very closely associated with TGF-$\beta$ or M-CSF such that TGF-$\beta$ or M-CSF is embedded, partially or fully, in a liposome. It is to be understood that, at any degree of association between TGF-$\beta$ or M-CSF and the outer surface of the liposome, the association is substantially without entrapping the TGF-$\beta$ or M-CSF entirely within the aqueous layer of the liposome.

It is also to be understood that the association between TGF-$\beta$ or M-CSF and liposome is accomplished through means other than covalent binding. Noncovalent binding is advantageous in that, first, the maximum biological activity of the bioactive agent is required and, second, the association is achieved by simple means. In addition, noncovalent binding is advantageous in that the associated peptide can more readily dissociate from the liposome.

The association of liposomes and peptides is advantageous for several reasons. Liposomes exhibit prolonged circulation so that liposome-associated agents will circulate in the bloodstream longer than most free drugs. In fact, the time constants reported for clearance from the system range from minutes to more than a day. Longer times are reported for small vesicles made with distearoylphosphatidylcholine and sphingomyelin. J.N. Weinstein, "Liposomes in the Diagnosis and Treatment of Cancer," in Liposomes From Biophysics to Therapeutics, ed. Marc J. Ostro (1987).

In addition, association of the bioactive agents with liposomes may increase the bioavailability of the agent. The solid phase vesicles of the present invention tend to absorb to cell surfaces more than fluid vesicles, thereby enhancing presentation of the agent to the target cell.

A preferred embodiment of the present invention makes use of the ability of negatively-charged liposomes to be taken up by membrane receptors on a cell surface. Thus, these liposomes can be effective for delivery into non-phagocytic cells as well as phagocytic cells.

Another advantage of the present invention is that association of bioactive agents with liposomes may protect the bioactive agent from host enzymes and other physiological clearing mechanisms. Such protection will enhance the bioavailability of the agent.

In the present invention, the aggregates formed of both TGF-$\beta$ with liposome and M-CSF with liposome exhibited biological activity. As used herein, biological activity refers to the ability of the bioactive agent to inhibit, promote or otherwise affect cellular mechanisms. As discussed above, in certain circumstances, TGF-$\beta$ exhibits proliferative and antiproliferative effects, as well as effects that are unrelated to proliferation. Although the biological activity of TGF-$\beta$ can be tested by other assays, the ability of TGF-$\beta$ to inhibit cell growth is preferred as an indicator of biological activity. Specifically, the biological activity of TGF-$\beta$ can be determined using the mink lung epithelial cell assay described in Cone et al., supra. This assay is based on the observation that the monolayer growth of the mink lung epithelial cells is inhibited in the presence of TGF-$\beta$. Details are provided in Example 3 below.

Similarly, as discussed above, the biologic activity of M-CSF refers to the ability of M-CSF to inhibit, promote or otherwise affect cellular mechanisms. Although the activity of the M-CSF of the invention may be determined in several ways, the ability of M-CSF to increase the proliferation of cells is preferred. Specifically, the biological activity of M-CSF can be determined using C3H/HeJ murine bone marrow cells in the assay as described in Strassmann, G., et al., "Regulation of Colony-Stimulating Factor 1-dependent Macrophage Precursor Proliferation by Type $\beta$ Transforming Growth Factor", Journal of Immunology 140 (8):2645-2651 (1988).

The term pharmaceutically effective amount, as used herein, shall mean that amount of a bioactive agent that is effective to inhibit, promote, or otherwise affect cellular mechanisms. The term thus includes effective amounts to treat conditions such as, but not limited to, rheumatoid arthritis, systemic lupus erythematosis, autoimmune diabetes, autoimmune thyroiditis, multiple sclerosis, and other diseases. Bioactive agents may also be used to stimulate effector functions of mature monocytes such as antifungal activity and lymphokine-induced tumoricidal activity. In addition, the bioactive agents may be used in the treatment of diseases that require elevation in blood monocytes, such as leukopania. Other conditions for which bioactive agents can be used include the facilitation of allograft transplantation or the inhibition of tumor cells.

Persons of ordinary skill in the art would be able to determine the dosage of the biologically active agents of the instant invention using techniques that are known in the art. Those techniques are set out, for example, on pages 19-28 of Goodman and Gilman, The Pharmacological Basis of Therapeutics, pp. 5th Ed. (1975). Dosages can be ascertained through the use of the established assays and conventional dose-response studies. Further refinements of the calculations necessary to determine the appropriate dosage for treatment are routinely made by those of ordinary skill in the art and are within the array of tasks routinely performed by them without undue experimentation.

Appropriate pharmaceutically acceptable carriers, diluents and adjuvants can be used together with the compounds described herein to prepare the desired compositions for use in the treatment of patients. The pharmaceutical compositions of this invention will contain the biologically active compound together with a solid or liquid pharmaceutically acceptable nontoxic carrier. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, magnesium carbonate, magnesium stearate, sodium stearate, glycerol monostearate, talc, sodium chloride, dried skim milk, glycerol propylene glycol, water, ethanol and the like. These compositions can take the form of solutions, suspensions, tablets, pills, capsules, powders, sustained-release formulations and the like. Suitable pharmaceutical carriers are described in "Remington's Pharmaceutical Sciences" by E.W. Martin. Such compositions will contain an effective therapeutic amount of the active compound together with a suitable amount of carrier so as to provide the form for proper administration to the patient. While intravenous injection is a very effective form of administration, other modes can be employed, such as by injection, or by oral or parenteral administration.

The compounds of the present invention can be used in the treatment of patients. Patients shall be used in its broadest sense to mean mammals, including humans, as well as laboratory animals, for example, dogs, cats, guinea pigs, mice, and rats.

The invention will be further clarified by the following examples, which are intended to be purely exemplary of the invention.

## EXAMPLE 1

### PREPARATION OF LIPOSOMES, TGF-$\beta$ AND TGF-$\beta$ LIPOSOME AGGREGATES

#### A. Liposome Preparation

Small unilamellar vesicle type liposomes were prepared using Egg L-Alpha-Lecithin (Phosphatidylcholine; PC) and brain Phosphatidyl Serine (PS), purchased from Avanti Polar Lipids, Inc. (Pelham, Alabama), and radio labelled phosphatidylcholine, L-Alpha-[Dipalmitoryl-1$^{14}$C] - Phosphatidyl Choline (107m Ci/m Mol), purchased from New England Nuclear. Neutral liposomes were prepared using PC alone. Negatively charged liposomes were prepared by mixing PC and PS at 1:1 molar ratio.

Both PC and PC/PS lipid components were mixed with chloroform and dried to a thin film under a stream of nitrogen. Next, the two mixtures were subjected to a vacuum for one hour to remove residual traces of organic solvent. Both mixtures were then hydrated by the addition of phosphate buffered saline (PBS) (without Ca$^{+2}$ and Mg$^{+2}$) at a pH of 7.2, vortex shaken for five minutes and allowed to swell for thirty minutes at room temperature. Thereafter, both mixtures were sonicated in a bath sonicator supplied by Laboratory Supplies, Hicksville, New York, for one hour or until no further optical clearance of the solution could be observed. The liposome preparations were then sterilized by filtration through a 0.2 $\mu$m (micron) polycarbonate filter. In some experiments, a trace amount of radio-labelled PC, dipalmitoyl $^{14}$C phosphatidyl choline, from Dupont, was included to form radiolabelled liposomes of both types.

#### B. The Preparation of Transforming Growth Factor

TGF-$\beta$ was purified from outdated human platelets according to a modification of the method described in Cone, et al. "An Improved Method of Purification of Transforming Growth Factor, Type Beta from Platelets," Analytical Biochemistry 168: 71-74 (1988). Following the last step of purification recited in the above reference, TGF-$\beta$ in acetonitrile was aliquoted into twice siliconized glass tubes and kept frozen until use.

#### C. Association of TGF-$\beta$ to Liposome

The twice-siliconized glass tube containing TGF-$\beta$ were lyophilized for fifteen minutes to reduce acetonitrile levels. Both types of liposome preparations, neutral and negatively-charged, were added for a total volume of 0.2 ml at various lipid to protein ratios. In some experiments, a trace amount of radioactive TGF-$\beta$, $^{125}$I-TGF-$\beta$, obtained from Biomedical Technologies, Inc. (Stoughton, MA) was included. Both mixtures were then incubated for one hour at 4°C with gentle agitation.

## EXAMPLE 2

### CHARACTERISTICS OF TGF-$\beta$-LIPOSOME AGGREGATES

#### A. Selection of Lipid:Protein Ratio

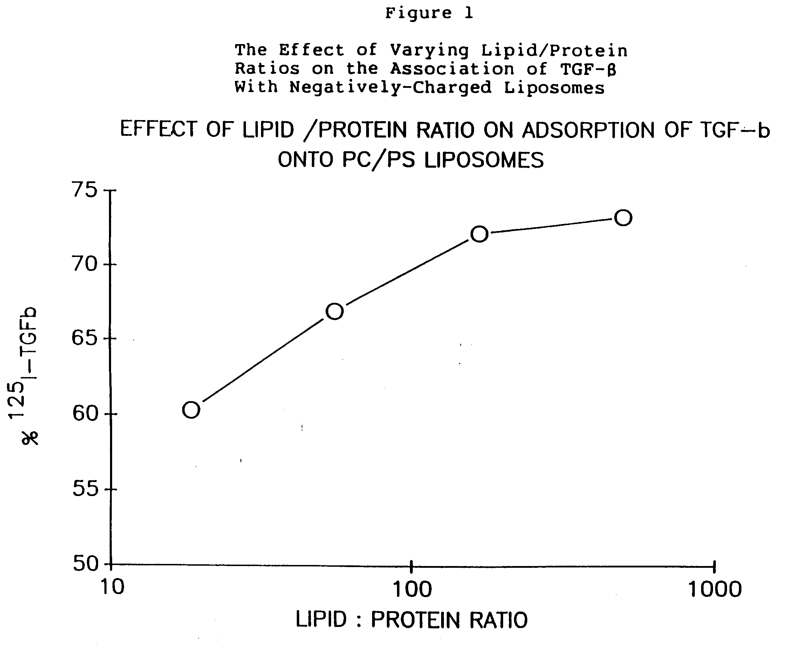

To determine what ratio of lipid to protein ratio yielded the maximum association of TGF-$\beta$ to liposome, lipid to protein ratios of 18:1, 55:1, 167:1 and 500:1 on a w/w basis were used. As set forth in Fig. 1, the percent $^{125}$I-TGF-$\beta$ present in the aggregates increased from 60% at a ratio of 18:1 up to approximately 71.5% at the ratio of 167:1. At lipid to protein ratios of higher than 167:1, no significant increase in the association of TGF-$\beta$ to liposome could be observed. Accordingly, the ratio 500:1 was selected for use in associating liposomes with TGF-$\beta$.

B. Efficiency and Conditions of Association

To determine the efficiency and the conditions of binding between TGF-$\beta$ and the liposomes, preparations with lipid:protein ratios of 500:1 were centrifuged in a Beckman ultra centrifuge using a SW-60 rotor, at 38,000 RPM for sixteen hours in discontinuous sucrose gradients.

Radioactivity was measured in a gamma counter for the presence of $^{125}$I. The activity of TGF-$\beta$ was determined by an inhibition assay using mink lung epithelial cells (CCL64) as indicators as described in Cone et al. supra.

As set forth in Table 1, migration patterns of $^{125}$I-TGF-$\beta$ indicated association of TGF-$\beta$ with PC/PS liposome. The migration pattern of $^{125}$I-TGF-$\beta$ in preparations of neutral PC liposomes was similar to that exhibited by $^{125}$I-TGF-$\beta$ in preparations where no lipids were present (category designated "NONE"), indicating that, rather than associating and migrating with neutral PC liposomes, TGF-$\beta$ migrated throughout the gradient as an unassociated protein. In contrast, when negatively-charged PC/PS liposome preparations were employed, TGF-$\beta$ exhibited a tight migration pattern and migrated into the 5%, 10%, and 20% levels. The presence of biologically active TGF-$\beta$ at each of those levels was determined by mink lung epithelial cell (CCL-64) assay to be the greater than 94.5 ng. In contrast, no activity level greater than 21 ng was found in any band in the gradients using TGF-$\beta$ and PC liposomes. These results suggest that the presence of $^{125}$I-TGF-$\beta$ corresponded to increased TGF-$\beta$ activity in the PC/PS preparation.

In addition to exhibiting a tight migration pattern in a discontinuous sucrose gradient, TGF-$\beta$ associated with PC/PS liposomes exhibited very little non-specific binding to the ultracentrifuge tube compared to TGF-$\beta$ with PC liposomes or TGF-$\beta$ alone. Specifically, as set forth in the lower part of Table 1, only 8% of $^{125}$I-TGF-$\beta$ in presence of PC liposomes or TGF-$\beta$ alone. Thus, the adherance of the TGF-$\beta$ molecules appeared to be greatly diminished when associated with PC/PS liposomes.

TABLE I

| DISTRIBUTION OF TGF-BETA ASSOCIATED WITH LIPOSOMES IN DISCONTINUOUS SUCROSE GRADIENT | | | | | |
|---|---|---|---|---|---|
| LIPID COMPOSITION | | | PC/PS | | PC | NONE |
| % SUCROSE | %$^{125}$I-TGF-$\beta$ | ACTIVITY (ng) | %$^{125}$I-TGF-$\beta$ | ACTIVITY (ng) | %$^{125}$I-TGF-$\beta$ |
| 0 | 4.3 | 2.27 | 4.5 | 6.8 | 7.8 |
| 5 | 9.2 | 94.5 | 8.8 | 9.45 | 18.0 |
| 10 | 26.5 | 94.5 | 10.1 | 20.5 | 15.2 |
| 20 | 41.5 | 94.5 | 12.6 | 21 | 12.2 |
| 40 a | 4.4 | 21.0 | 8.8 | 21 | 3.6 |
| 40 b | 4.4 | 15.0 | 8.8 | 19.95 | 3.6 |
| IN ULTRA CENTRIFUGE-TUBE | 8.0 | N.D. | 41 | N.D. | 40 |

\* LIPID TO PROTEIN RATIO 500 : 1.
38000 RPM, 16 HOURS in SW-60 ROTOR

C. Distribution of TGF-$\beta$ Liposomes on Sucrose Gradients

In a second set of experiments, a continuous sucrose gradient was used according to the method of Baxter-Baggard, "A Simple Method for the Large-Scale Preparation of Sucrose Gradients," FEBS Letters, 20(1): 117-119 (January 1972). The gradient was obtained by subjecting 20% sucrose in PBS to three rounds of freezing and thawing. Approximately 300 $\mu\ell$ of TGF-$\beta$ liposome were loaded onto four ml gradients which were then centrifuged at 100,000 times g at 4° centigrade for eighteen hours in the ultra centrifuge.

At the end of the centrifugation, 0.2 ml fractions were collected from the top the gradient. Radioactivity was measured by liquid scintillation counter for $^{14}$C and in a gamma counter for $^{125}$I. The amount of biologically active TGF-$\beta$ in each fraction was determined by inhibition assay using mink lung epithelial cells as indicators and known amounts of TGF-$\beta$ as a standard, according to the method described in Cone, et al., supra.

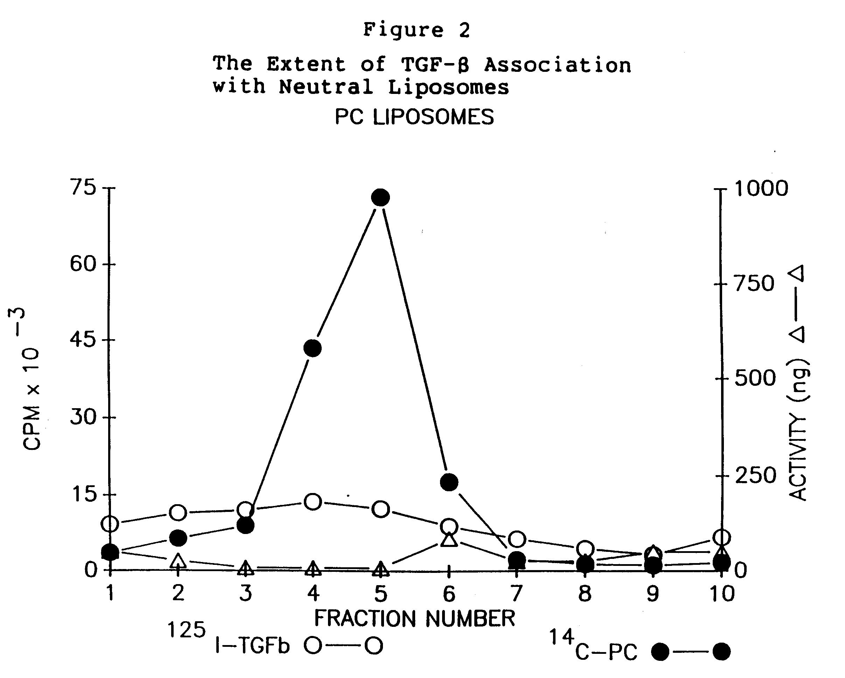

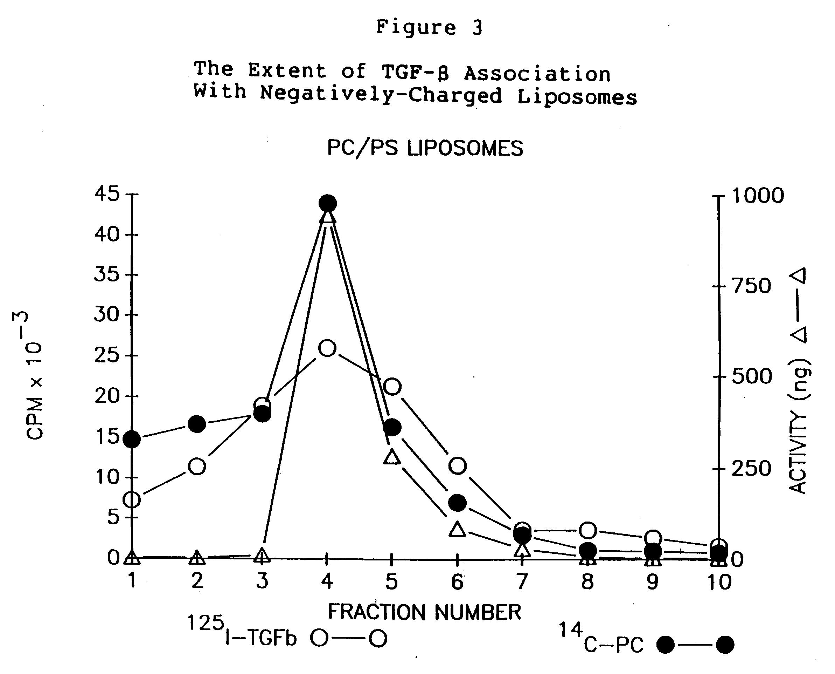

Figures 2 and 3 demonstrate the association of TGF-$\beta$ to liposome preparations made up of PC/PS lipids and lack of association of TGF-$\beta$ to liposome preparations made up of only PC liposomes.

In both figures, three measurements were made for each fraction:

$^{14}$C-PC - indicating presence of PC component of liposomes;

$^{125}$I-TGF-$\beta$ - indicating presence of TGF-$\beta$; and

TGF-$\beta$ activity - confirming presence of TGF-$\beta$ and indicating biological activity of TGF-$\beta$.

In Figure 2, where only PC is employed as liposome constituent, presence and activity of TGF-$\beta$ were independent of presence of liposomes. Throughout all ten fractions, presence of TGF-$\beta$ ranged from about 10 to 14 cpm x $10^3$ and activity of TGF ranged from 0 to 75 ng/fraction. In contrast, in Figure 3, where negatively-charged PC/PS liposomes were employed, both presence of TGF-$\beta$ and activity of TGF-$\beta$ were highly correlated to presence of PC/PS liposomes. Specifically, fraction 4, which contained the highest level of negatively-charged liposomes, also contained highest level of TGF-$\beta$, 25 x $10^3$ CPM, and exhibited greatest TGF-$\beta$ activity, approximately 925 ng. Accordingly, it is demonstrated that TGF-$\beta$ associates with negatively charged PC/PS liposomes and not with neutral PC liposomes.

## EXAMPLE 3

## DETERMINATION OF BIOLOGIC ACTIVITY IN VITRO

Because TGF-$\beta$ is known to suppress the stimulation of mink lung epithelial cells, designated MV-1-LU cells (American Type Culture Collection CCL-64 cells (hereinafter CCL-64 cells)) that action was employed to determine biologic activity in vitro. Specifically, the ability of TGF-$\beta$ and TGF-$\beta$ associated liposomes were analyzed for the ability to inhibit proliferation of these cells. The extent of inhibition was determined by tritiated thymidine incorporation.

Mink lung epithelial cells (American Type Tissue Collection CCL64) maintained in Dulbecco's modified Eagles' medium (GIBCO), ("DMEM") plus 10% fetal calf serum (HyClone) are known to be released by trypsin. 0.1 ml of cells containing 2 x $10^4$ ml were seeded into 96-well tissue culture plates. Aliquots of TGF-$\beta$ standard or samples to be assayed were then added to the plates. After a 24 hour incubation at 37°C ub a 5% $CO_2$ atmosphere, the wells were pulsed with 25 $\mu$l of 40 $\mu$Ci/ml tritiated thymidine (New England Nuclear, 6.7 Ci/mM). Approximately 16 hours later, the wells were washed three times with DMEM and incubated for 15-20 minutes with trypsin to release the cells. The cells were then collected on glass paper by a semi-automated cell harvester (Skatron). Using the same bioassay, a standard curve with known amounts of TGF-$\beta$ was constructed. The amount of active TGF-$\beta$ in a given sample was determined by comparison of the inhibition obtained to the standard curve.

As set forth in Table II, the percentage inhibition was measured for three treatments: PC/PS liposomes alone, for TGF-$\beta$ associated with PC/PS liposomes, and for TGF-$\beta$ alone. As set forth in the Constant Presence portion of Table II when the three solutions were aliquoted directly into the tissue culture plates and kept at a constant presence, both TGF-$\beta$ and TGF-$\beta$ associated with liposomes exhibited high percentages of inhibition, 82% and 90%, respectively, in contrast to 0% inhibition by liposomes alone. Thus, the biological activity of TGF-$\beta$ associated with liposomes corresponds to the biological activity of TGF-$\beta$ alone.

To test the effect of association of TGF-$\beta$ with liposome on absorption by cells, the cells were then subjected to sequential transfer. In this study, identical cultures of cells exposed to the three treatments were sequentially transferred at thirty minute intervals into fresh tissue culture plates.

The cells that were exposed to liposome alone exhibited no inhibition after each transfer. The pattern of inhibition varied, however, for cells that were exposed to TGF-$\beta$ with and without liposomes. For cells exposed to TGF-$\beta$ alone, the cells of the first transfer exhibited essentially the same inhibition as that demonstrated with constant presence. That inhibition decreased to a significant degree, however, with the second transfer. Specifically, cells exposed to only TGF-$\beta$ exhibited a percentage inhibition of 83% in the first transfer but only 35% in the second transfer. In the third, fourth, fifth, and end transfer, the cells exhibited no inhibition. Accordingly, it appeared that the TGF-$\beta$ molecules were absorbed by the cells in the first and second transfer so that the cells of the later transfers did not contain enough TGF-$\beta$ to show any anti-proliferative effects.

In contrast, the cells that were exposed to TGF-$\beta$ associated with liposomes exhibited another pattern of inhibition. Although the cells of the first transfer exhibited an inhibition of 54%, this may be attributed to leakage of TGF-$\beta$ from the cells. The significance lies in the inhibition exhibited by the cells of the end transfer. That percentage of inhibition, 81%, at the end of transfer is similar to the inhibition seen with cells exposed to a constant presence of TGF-$\beta$. Accordingly, it appeared that the liposomes inhibited adherance

of TGF-$\beta$ to the cells such that TGF-$\beta$ was sequentially transferred and able to exhibit its antiproliferative effect in the end transfer.

TABLE II

| TGF-$\beta$ LIPOSOMES DELIVER A NON-ABSORBABLE NEGATIVE SIGNAL FOR SERUM STIMULATED CCL-64 | | | |
|---|---|---|---|
| PERCENTAGE OF INHIBITION | | | |
| TREATMENT | LIPOSOMES | TGF-$\beta$ LIPOSOMES (1.25ng/ml) | TGF-$\beta$ (1.25ng/ml) |
| CONSTANT TRANSFER | 0 | 90 | 82 |
| FIRST TRANSFER (30 MIN) | 0 | 54 | 83 |
| SECOND TRANSFER (30 MIN) | 0 | 0 | 35 |
| THIRD TRANSFER (30 MIN) | 0 | 0 | 0 |
| FOURTH TRANSFER (30 MIN) | 0 | 0 | 0 |
| FIFTH TRANSFER (30 MIN) | 0 | 0 | 0 |
| END TRANSFER | 0 | 81 | 0 |
| CONTROL = 57081 CPM | | | |

EXAMPLE 4

THE ABILITY OF TGF-$\beta$-LIPOSOME AGGREGATES TO SUPPRESS THE IMMUNE REACTION AGAINST C. PARVUM IN VIVO.

Because the spleen functions to filter blood, bacterial infection often leads to nonspecific acute splenitis which results in an enlargement of the spleen and an increase in the number of lymphocytes within the spleen. The ability of TGF-$\beta$ to inhibit proliferation of spleen cells otherwise stimulated by bacterial infection was employed as an in vivo assay for the activity of TGF-$\beta$ associated with liposomes.

In this assay, the strain of mice known as C57BL/6 were inoculated intraperitonially with 0.2 ml of PBS containing either 0.7 or 1 mg heat-killed Corynobacterium parvum (obtained from Welcome Research Laboratories, Research Triangle Park, North Carolina). Twenty-four hours later, the mice received an intravenous injection of 0.2 ml of PBS containing (a) 1 $\mu$g of TGF-$\beta$, (b) 0.5 mg PC/PS liposome, or (c) 1 $\mu$g of TGF-$\beta$ associated with 0.5 mg PC/PS liposomes (1:500 ratio). These solutions were not subjected to sucrose gradients. The treatment was repeated three times at daily intervals.

Six to eight days after immunization with bacteria, the mice were sacrificed. The spleens were weighed and single cell suspensions were analyzed to determine the extent of the immune response against the bacteria. The results are presented in Table III.

Based on the anti-proliferative properties of TGF-$\beta$, it was expected that spleens of mice that received 1 $\mu$g TGF-$\beta$ associated with 0.5 mg PC/PS liposomes would not exhibit either increased size or cell number. In contrast, it was expected that spleens of mice that received either only 1 $\mu$g TGF-$\beta$ or 0.5 mg PC/PS liposomes would exhibit the enlarged spleen cell and increased spleen cell number of an advanced immune response.

In Experiment I, mice that had been treated with TGF-$\beta$ associated with liposomes yielded a mean spleen weight of 109 ± 12 milligrams and 232 x $10^6$ cells per spleen. These data corresponded to an

10

inhibition of the immune response of 82%. In contrast, mice that had received only TGF-$\beta$, without liposomes, exhibited spleen weights of 165 ± 30 milligrams and 308 x $10^6$ cells per spleen for an inhibition of 44%. Mice treated with only liposomes exhibited spleen weights of 224 ± 10 milligrams and 440 x $10^6$ cells per spleen, which indicated percent inhibition of only 10%.

In Experiment II, mice which had received TGF-$\beta$ associated with liposomes yielded spleen weights of 139 ± 18, and 227 x $10^6$ cells per spleen, which indicated an inhibition of 92%. This figure is nearly three times that of TGF-$\beta$ injected mice where spleen weights of 172 ± 57 milligrams and cell numbers of 342 x $10^6$ cells per spleen were obtained for an inhibition rate of 36%.

## TABLE III

### EFFECT OF TGF-BETA ASSOCIATED WITH LIPOSOMES ON ACUTE SPLENITIS IN C. PARVUM CHALLENGED C578L/6 MICE

| INJECTION OF C. PARVUM | TREATMENT | SPLEEN WEIGHT (MG)* | # CELLS PER SPLEEN ( x $10^6$) | % INHIBITION |
|---|---|---|---|---|
| | | EXPERIMENT I | | |
| - | - | 72 ± 3 | 179 | - |
| + | PBS | 239 ± 103 | 466 | - |
| + | LIPOSOMES | 224 ± 10 | 440 | 10 % |
| + | TGF-BETA | 165 ± 30 | 308 | 44 % |
| + | TGF-BETA & LIPOSOMES | 109 ± 18 | 227 | 92 % |
| | | EXPERIMENT II | | |
| - | - | 106 ± 33 | 211 | - |
| + | LIPOSOMES | 210 ± 30 | 417 | - |
| + | TGF-BETA | 172 ± 57 | 342 | 36 % |
| + | TGF-BETA & LIPOSOMES | 139 ± 18 | 227 | 92 % |

*   AVERAGE OF 3 MICE ± S.D.

EXAMPLE 5

DELAYED TYPE HYPERSENSITIVITY (DTH) TO LISTERIA MONOCYTOGENESIS

As described in Kaufmann and Hahn, "Biological Functions of T-Cell Lines with Specificity for the Intracellular Bacterium Listeria monocytogenes In-Vitro and In-Vivo," J. Exp. Med. 155: 1754-1765 (June 1982), experimental infection of mice with Listeria monocytogenesis has been widely used to study cellular immune response to bacteria. In a double blind study, the strain of mice known as C57B1/6 were injected with live bacteria at a concentration of 5 x $10^4$. Five or six days later, the spleens of those mice were removed and used to prepare a single cell suspension. After removal of adherent cells, the nonadherent splenocytes at a concentration of $25 \times 10^6$ were injected intravenously into syngeneic recipient mice, i.e., mice that were genetically identical to the initial injected mice with respect to immune reactions. Sixteen hours later, these later injected mice were divided into three or four groups. One group received 0.2 ml PBS containing 1 $\mu$g TGF-$\beta$; another received PBS with 0.5 mg PC/PS liposome, the third group received 1 $\mu$g TGF-$\beta$ associated with 0.5 mg PC/PS liposomes (1:500 ratio), and the fourth, where present, received 0.2 ml PBS alone.

One hour later, the right foot pad of each mouse was injected with 50 ul of PBS containing $5 \times 10^7$ heat-killed <u>Listeria</u>, and, as a control, the left foot was injected with 50 $\mu$l PBS. Foot pad swelling was measured 24 hours later with a micrometer. A 0.01 mm difference in thickness between the <u>Listeria</u>-injected and PBS injected foot pad was taken as one DTH unit.

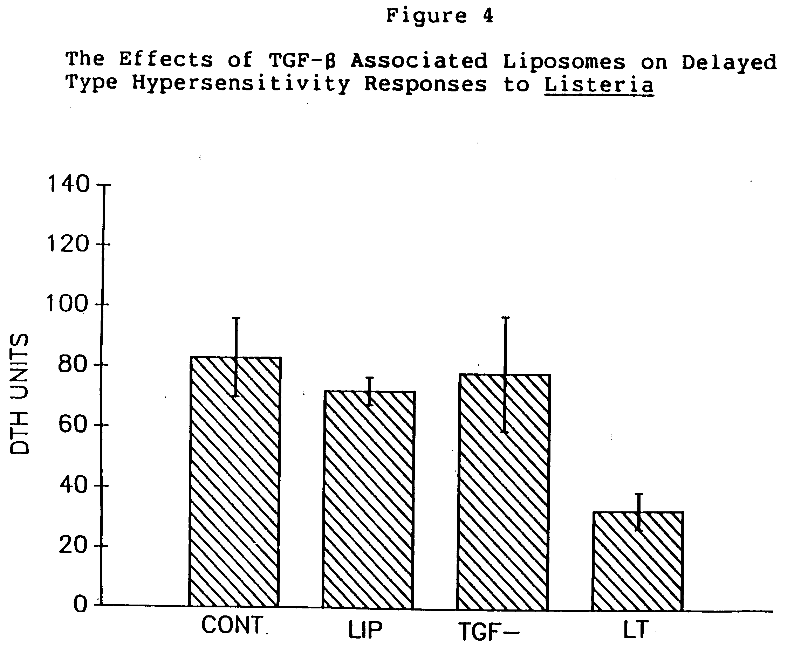

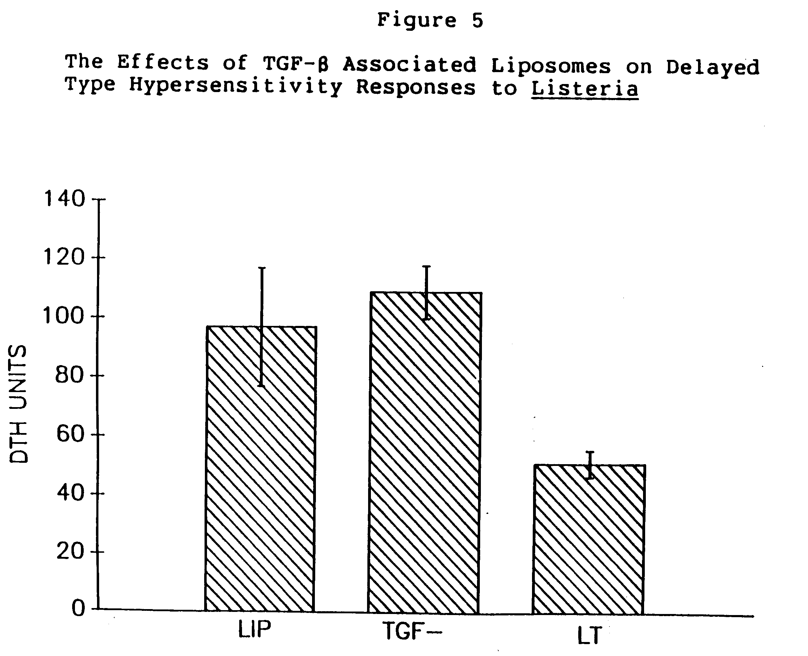

The difference in foot pad swelling, as indicated by DTH units, corresponds to the effect of TGF-$\beta$ on the immune response. Specifically, the antiproliferative effect of TGF-$\beta$ manifests as decreased foot pad swelling and fewer DTH units. As set forth in Figures 4 and 5, the presence of liposomes associated TGF-$\beta$ caused a significant decrease in foot swelling. Specifically, compared to liposomes alone (LIP), TGF-$\beta$ along (TGF-$\beta$), or a control (CONT), TGF-$\beta$ associated with liposomes (LT) exhibited significantly fewer DTH units. In both Figs. 4 and 5, the decrease in DTH units for TGF-$\beta$ liposomes (LT), represents a 50% reduction in the immune response with a statistical difference of ($P < 0.01$). Accordingly, TGF-$\beta$ associated with liposomes exhibited in vivo reduction in the immune response.

EXAMPLE 6

PREPARATION OF LIPOSOMES, M-CSF, AND M-CSF LIPOSOME AGGREGATES

A. Positively-Charged Liposome Preparation

Small unilamellar vesicle type liposomes were prepared using 10 mg of Egg L-Alpha-Lecithin (Phosphatidylcholine; PC) purchased from Avanti Polar Lipids, Inc. (Pelham, Alabama), 0.5 $\mu$Ci radio labelled phosphatidylcholine, L-Alpha-[Dipalmitoryl-114C] - Phosphatidyl Choline (107m Ci/m Mo1), purchased from New England Nuclear, and 2.5 mg of stearylamine purchased from Sigma.

Neutral liposomes (for controls) were prepared as above except without stearylamine.

The lipid components were mixed with chloroform in a glass tube and dried to a thin film under a stream of nitrogen. Next, the two mixtures were lyophilized for 30 minutes to remove residual traces of organic solvent. Both mixtures were then hydrated by the addition of phosphate buffered saline (PBS) (without Ca+2 and Mg+2) to a concentration of 5 mg/ml liquid at a pH of 7.2, vortex shaken for five minutes and allowed to swell for thirty minutes at room temperature. Thereafter, both mixtures were sonicated in a bath sonicator supplied by Laboratory Supplies, Hicksville, New York, three times, at approximately 20 minutes each time, at room temperature. The liposome preparations were then sterilized by filtration through a 0.2 $\mu$m (micron) polycarbonate filter.

B. The Preparation of Macrophage Colony-Stimulating Factor

The M-CSF was obtained by the renaturation and purification of human truncated M-CSF expressed in E. coli.

1. Construction and Expression

The expression of a truncated form of human M-CSF was performed using two cistronic expression systems. The COS expression plasmid designated pcDhMCSF11-185, which encodes N-terminal 185 amino acid residues of the 554 amino acid M-CSF precursor was digested with Scal and BamHi restriction enzymes. The resultant fragment (about 450 bp) was ligated with a synthetic linker which possessed an internal SD sequence, a termination codon for the first cistron and an initiation codon for the second cistron. The ligated fragment was inserted between the Xbal and BamHi sites of prepIL-2D8 . The resultant plasmid, ptrpII-2X M-CSF101, coding for a 151 amino acid sequence of M-CSF was then transformed into E. coli by the CaCl2 method. Such recombinant techniques are provided in the copending U.S. Application Serial No. 07/304,692, filed February 1, 1989, entitled "Human Colony-Stimulating Factors," to Takahashi, M. et. al.

2. Renaturation

The transformed E. coli cells were shaken in supplemented M9 medium which also contained ampicillin. The cells were harvested and pelleted and washed with Triton X-100. The final pellet contained an inclusion body containing the truncated M-CSF. The pellet was solubilized in 7.0 M guanidin hydrochrolide and 25mM 2-mercaptoethanol with stirring for 4 hours at room temperature. The solubilized pellet was slowly dropped into 2,000 ml of glutathione solution (0.5 mM reduced, 0.1 mM oxidized glutathione and 2.0 M urea in 50 mM Tris/HCL, ph 8,5) with intense stirring. The solution was kept at 4°C for 48 hours.

After 10 ml of the renatured solution was concentrated to 500ul in an Amicon membrane, the solution was applied at a rate of 0.7 ml/min to a gel filtration HPLC on Shodex WS-803 column previously equilibrated with 40 mM sodium phosphate containing 0.3 M NaCl, ph 6.8. The fractions were collected and assayed for M-CSF activity.

3. Purification

The renatured solution was centrifuged and the supernatant applied to QAE-ZeTA Prep Cartridge 100 from Pharmacia-LKB which was previously equilibrated with 50 mM Tris/HCl at ph 8.5 and eluted with 0.5 M NaCl in 50 mM Tris/HCl, pH 8.5.

After ammonium sulfate precipitation, the supernatant was applied to a TSK-gel Phenyl-5PW HPLC column (Toso, 21.5 x 1500 mm), preequilibrated with 40 mM sodium phosphate, pH 7.4 and containing a saturated ammonium sulfate solution. The active fragments were obtained at 6-3% ammonium sulfate and concentrated and exchanged against 40 mM sodium phosphate, pH 7.4.

The concentrated sample was applied to a TSK-gel DEAE 5PW column (Toso, 21.5 x 150 mm) preequilabrated with 40 mM sodium phosphate, pH 7.4, and eluted at a flow rate of 3 ml/min by a NaCl gradient.

C. Preparation of Liposome M-CSF Aggregates

The combinations of liposome and M-CSF set forth in Table VI below were mixed together, vortexed, incubated at room temperature for 5 minutes.

TABLE VI

| Addition Tube # | $C^{14}$-PC/S Liposomes | $C^{14}$-PC Liposomes | M-CSF | $^{125}$I-M-CSF |
|---|---|---|---|---|
| 1 | 500 ^ mgm (100 ^ mli) | -- | 5ugm (5 ^ mli) | 0.1 ^ mgm (6X10)$^5$ cpm in |
| 2 | 500 ^ mgm (100 ^ mli) | -- | 5ugm (5 ^ mli) | -- |
| 3 | 500 ^ mgm (100 ^ mli) | -- | -- | -- |
| 4 | -- | 500 ^ mgm | 5 ^ mgm | 0.1 ^ mgm |
| 5 | -- | 500 ^ mgm | 5 ^ mgm | -- |
| 6 | -- | 500 ^ mgm | -- | -- |
| 7 | -- | -- | 5 ^ mgm | 0.1 ^ mgm |

EXAMPLE 7

CHARACTERISTICS OF M-CSF-LIPOSOME AGGREGATES

A. Preparation of Sucrose Gradients

To form sucrose gradients, 1 ml of a 20% sucrose solution was added to the bottom of polyallomer tubes 1-7. On the top of each layer, 1 ml aliquots of 10% and 5% and 2.5% sucrose were carefully layered to form final volumes of 4 ml in each of the tubes.

The liposome-M-CSF mixtures prepared in Example 6 (c) above were loaded on the top of the appropriately numbered sucrose gradient. The tubes were centrifuged in a Beckman ultracentrifuge for 16 hrs at 40000 rpm in SW-60 rotor. At the end of centrifugation, 0.2 ml fractions were collected from the top of the gradient and counted for radioactivity of 14C and 125I. The levels of 14C indicated the relative concentration of liposome in the fractions. Similarly, the level of 125I indicated the relative concentration of M-CSF in the fractions. After the fractions were filter sterilized, the activity of M-CSF in each fraction was determined against a M-CSF standard curve in the assay set forth below.

B. Assay for M-CSF Activity

The C3H/HeJ murine bone marrow cells which proliferate in response to M-CSF were used to assay the activity of M-CSF. The cells were seeded in flat-bottomed microtiter plates (Costar, Cambridge, Ma) at a density of 1 x 105. The mixtures of liposome and M-CSF were serially diluted and added to the plates for a final volume of 0.2 ml.

The cells were cultured for three days. Eight hours before the end of the culture period, cells were pulsed with 1μCi of [methyl-3H] TdR (6.7 Ci/mmol; New England Nuclear Boston, Ma). The incorporation of [methyl-3H] TdR into the cells was determined by standard liquid scintillation counting procedures after harvest of cells using a semiautomatic cell harvester (Skatron, Sterling, Va).

The extent of proliferation in a given fraction was extrapolated from M-CSF dose curves prepared under the same conditions. Strassman, G., et al., "Regulation of Colony-Stimulating Factor 1-dependent Macrophage Precursor Proliferation by Type $\beta$ Transforming Growth Factor", Journal of Immunology 140 (8)-:2645-2651 (1988); Moore, R.N., et al., "Endogenous Regulation of Macrophage Proliferative Expansion by Colony-Stimulating factor-induced Interferon," Science 223: 178 (1984); and Pullen, S.K. et al., "Bone Marrow-derived Macrophage Expression of Endogenous and Transfected Class II MHC Genes during Differentiation In Vitro," Journal of Immunology 137: 1359 (1986).

C. Efficiency and Conditions of Association

As set forth in Table VII below, the migration patterns of 125I indicate association of M-CSF with positively charged liposomes. The migration pattern of M-CSF in preparations of neutral PC liposomes (PC - M-CSF in Tubes 4 and 5) was similar to that exhibited by M-CSF in preparations without liposomes (M-CSF only in Tube 7), indicating that, rather than associating and migrating with neutral liposomes, M-CSF migrated throughout the gradient as an unassociated protein. In contrast, when positively-charged PC/S liposome preparations were employed (PC/S - M-CSF in Tubes 1 and 2), M-CSF exhibited a tight migration pattern and predominated in fractions 2-6, the same fractions into which PC/S liposomes predominated, both in the presence of M-CSF (Tubes 1 and 2) and in the absence of M-CSF (Tube 3, PC/S only).

This study also showed that biologically active M-CSF associated with positively-charged liposomes. The presence of biologically active M-CSF in each fraction was determined with C3H/HeJ murine bone marrow cells as described above. In preparations of M-CSF alone (Tube 7) and M-CSF with neutral PC (Tubes 4 and 5), fractions 6-9 contained biologically active M-CSF. The presence of neutral liposome seemed to have no impact on the presence of biologically active M-CSF. In contrast, in preparations of M-CSF with positively-charged liposomes (PC/C - M-CSF in Tubes 1 and 2), those fractions in which liposomes predominated were the same fractions in which both presence and activity of M-CSF were greatest. Thus, it appears that the presence of positively charged liposomes affected the migration of biologically active M-CSF and suggests that M-CSF exhibits preferential association with positively-charged liposomes.

Table VII

Association of M-CSF (Recombinant Human) with
Preformed Liposomes with Opposite Charge

| Tube # Fraction From Top | PC/S - M-CSF 14C (1) | 125I (2) | Activity (ug) | PC/S Only 14C (3) | Activity (ug) | PC - M-CSF 14C (4) | 125I (5) | Activity (ug) | PC Only 14C (6) | M-CSF Only 125I (7) | Activity (ug) | % Sucrose |
|---|---|---|---|---|---|---|---|---|---|---|---|---|
| 1 | 722 | 34851 | | 689 | N.D. | 34 | 12090 | | 58 | 9926 | | 2.5% |
| 2 | 4322* | 216658* | 0.4 | 5546* | | 29 | 14160 | | 29 | 9863 | | |
| 3 | 2401* | 1129926* | 1.6 | 2374* | N.D. | 49 | 21190 | | 110 | 9698 | | |
| 4 | 2322* | 116803* | | 2285* | | 63 | 26933 | | 153 | 10914 | | |
| 5 | 1275* | 112835* | | 999 | | 98 | 54933 | | 226 | 28858 | | |
| 6 | 1225* | 1333836* | | 362 | | 182 | 103423* | | 304 | 89006* | 0.4 | 5.0% |
| 7 | 603 | 1306666* | | 266 | | 281 | 160693* | | 666 | 118213* | | |
| 8 | 429 | 1209998* | | 261 | | 2180* | 140677* | 1.5 | 2711* | 123342* | 0.8 | |
| 9 | 322 | 83688 | | 203 | | 3106* | 110186* | | 3417* | 73695* | | |
| 10 | 182 | 50430 | | 142 | | 3833* | 49568 | | 5272* | 57940 | | |
| 11 | 113 | 33304 | | 130 | | 3821* | 23318 | | 2912* | 16416 | | 10% |
| 12 | 130 | 21857 | | 75 | | 2005 | 10289 | | 1544 | 6905 | | |
| 13 | 85 | 13073 | | 66 | | 581 | 5531 | | 1187 | 4703 | | |
| 14 | 84 | 7333 | | 95 | | 896 | 4319 | | 832 | 2402 | | |
| 15 | 29 | 5149 | | 29 | | 502 | 2125 | | 391 | 1201 | | |
| 16 | 36 | 3750 | | 98 | | 362 | 566 | | 255 | 714 | | 20% |
| 17 | 55 | 2372 | | 34 | | 220 | 388 | | 104 | 441 | | |
| 18 | 107 | 2012 | | 66 | | 92 | 306 | | 63 | 307 | | |
| 19 | 62 | 1050 | | 66 | | 50 | 204 | | 50 | 106 | | |
| 20 | 60 | 561 | | 60 | | 60 | 200 | | 49 | 107 | | |

Lipid Protein Ration 100:1 (500 ug lipid; 5 ug M-CSF Ec-80-I)
40000 rpm, 16 hrs, SW-60 rotor 19°C
N.D. Not Detected

*represents peak

Other embodiments of the invention will be apparent to those skilled in the art from a consideration of the specification and practice of the invention disclosed herein. It is intended that the specification and examples be considered as exemplary only, with a true scope and spirit of the invention being indicated by the following claims.

# EP 0 393 707 B1

**Claims**

1. The use of aggregates comprising TGF-$\beta$ or M-CSF as a bioactive agent in association with the outer surfaces of charged liposomes for the manufacture of a medicament;
   the association being such that the biological activity of the bioactive agent is not substantially diminished when the aggregates are administered in a pharmaceutically effective amount to a patient.

2. The use according to claim 1, wherein the liposomes are small unilamellar vesicles, large unilamellar vesicles, or multilamellar vesicles.

3. The use according to claim 2, wherein the liposomes are small unilamellar vesicles.

4. The use according to claim 3, wherein the small unilamellar vesicles comprise phosphatidyl choline and phosphatidyl serine.

5. The use according to claim 4, wherein the phosphatidyl choline and phosphatidyl serine are present in a molar ratio of 1:1.

6. The use according to claim 3, wherein the small unilamellar vesicles are comprised of phosphatidyl choline and stearylamine.

7. The use according to any preceding claim, wherein the bioactive compound is TGF-$\beta$ and the liposomes bear a net negative charge.

8. The use according to any of claims 1-6, wherein the bioactive agent is M-CSF and the liposomes bear a net positive charge.

9. Aggregates of a bioactive agent selected from TGF-$\beta$ or M-CSF in association with liposomes, wherein the aggregates comprise:
   (a) a multiplicity of charged liposomes having outer surfaces; and
   (b) a multiplicity of the bioactive agents having a charge that is complementary to the charge on the liposomes;
   wherein the bioactive agent is associated with the outer surfaces of the liposome such that biological activity of the bioactive agent is not substantially diminished when the aggregates are administered in a pharmaceutically effective amount to a patient.

10. Aggregates according to claim 9, wherein the liposomes are selected from small unilamellar vesicles, large unilamellar vesicles, and multilamellar vesicles.

11. Aggregates according to claim 10, wherein the liposomes are small unilamellar vesicles.

12. Aggregates according to claim 11, wherein the small unilamellar vesicles are comprised of phosphatidyl choline and phosphatidyl serine.

13. Aggregates according to claim 12, wherein the phosphatidyl choline and phosphatidyl serine are in a molar ratio of 1:1.

14. Aggregates according to claim 11, wherein the small unilamellar vesicles are comprised of phosphatidyl choline and stearylamine.

15. Aggregates according to any of claims 9-14, wherein the bioactive agent is TGF-$\beta$ and the liposomes are negatively charged.

16. Aggregates according to any of claims 9-14, wherein the bioactive agent is M-CSF and the liposomes are positively charged.

17. A pharmaceutical composition comprising:
   (a) a pharmaceutically effective amount of the aggregates according to any of claims 9-16, and

16

EP 0 393 707 B1

(b) a pharmaceutically acceptable carrier.

**Patentansprüche**

1. Verwendung von Aggregaten, enthaltend TGF-$\beta$ oder M-CSF als bioaktives Mittel in Assoziation mit den äußeren Oberflächen geladener Liposome, zur Herstellung eines Medikaments, wobei die Assoziation so beschaffen ist, daß die biologische Aktivität des bioaktiven Mittels nicht wesentlich vermindert ist, wenn die Aggregate in einer pharmazeutisch wirksamen Menge an Patienten verabreicht werden.

2. Verwendung gemäß Anspruch 1, worin die Liposome kleine unilamellare Vesikel, große unilamellare Vesikel oder multilamellare Vesikel sind.

3. Verwendung gemäß Anspruch 2, worin die Liposome kleine unilamellare Vesikel sind.

4. Verwendung gemäß Anspruch 3, worin die kleinen unilamellaren Vesikel Phosphatidylcholin und Phosphatidylserin enthalten.

5. Verwendung gemäß Anspruch 4, worin das Phosphatidylcholin und Phosphatidylserin in einem molaren Verhältnis von 1:1 vorliegen.

6. Verwendung gemäß Anspruch 3, worin die kleinen unilamellaren Vesikel aus Phosphatidylcholin und Stearylamin zusammengesetzt sind.

7. Verwendung gemäß jedem vorhergehenden Anspruch, worin die bioaktive Verbindung TGF-$\beta$ ist und die Liposome eine negative Nettoladung aufweisen.

8. Verwendung gemäß jedem der Ansprüche 1 bis 6, worin das bioaktive Mittel M-CSF ist und die Liposome eine positive Nettoladung aufweisen.

9. Aggregate eines bioaktiven Mittels, ausgewählt aus TFG-$\beta$ oder M-CSF, in Assoziation mit Liposomen, worin die Aggregate umfassen:
   (a) eine Vielzahl geladener Liposome mit äußeren Oberflächen; und
   (b) eine Vielzahl bioaktiver Mittel mit einer Ladung, die zur Ladung auf den Liposomen komplementär ist,
   worin das bioaktive Mittel mit den äußeren Oberflächen des Liposoms assoziiert ist, und zwar so, daß die biologische Wirksamkeit des bioaktiven Mittels nicht wesentlich vermindert ist, wenn die Aggregate in einer pharmazeutisch wirksamen Menge an Patienten verabreicht werden.

10. Aggregate gemäß Anspruch 9, worin die Liposome aus kleinen unilamellaren Vesikeln, großen unilamellaren Vesikeln und multilamellaren Vesikeln ausgewählt sind.

11. Aggregate gemäß Anspruch 10, worin die Liposome kleine unilamellare Vesikel sind.

12. Aggregate gemäß Anspruch 11, worin die kleinen unilamellaren Vesikel aus Phosphatidylcholin und Phosphatidylserin zusammengesetzt sind.

13. Aggregate gemäß Anspruch 12, worin das Phosphatidylcholin und Phosphatidylserin in einem molaren Verhältnis von 1:1 vorliegen.

14. Aggregate gemäß Anspruch 11, worin die kleinen unilamellaren Vesikel aus Phosphatidylcholin und Stearylamin zusammengesetzt sind.

17

EP 0 393 707 B1

**15.** Aggregate gemäß jedem der Ansprüche 9 bis 14,
worin das bioaktive Mittel TGF-$\beta$ und die Liposome negativ geladen sind.

**16.** Aggregate gemäß jedem der Ansprüche 9 bis 14,
worin das bioaktive Mittel M-CSF und die Liposome positiv geladen sind.

**17.** Pharmazeutische Zusammensetzung, enthaltend:
(a) eine pharmazeutisch wirksame Menge der Aggregate gemäß jedem der Ansprüche 9 bis 16 sowie
(b) einen pharmazeutisch zulässigen Träger.

## Revendications

**1.** Utilisation d'agrégats contenant du TGF-$\beta$ ou du M-CSF comme agent bioactif, en association avec les surfaces extérieures de liposomes chargés, pour la fabrication d'un médicament;
l'association étant telle que l'activité biologique de l'agent bioactif n'est pas essentiellement diminuée lorsque les agrégats sont administrés en quantité pharmaceutiquement actives à un patient.

**2.** Utilisation selon la revendication 1, dans laquelle les liposomes sont de petits vésicules unilamellaires, de grands vésicules unilamellaires ou des vésicules multilamellaires.

**3.** Utilisation selon la revendication 2, dans laquelle les liposomes sont de petits vésicules unilamellaires.

**4.** Utilisation selon la revendication 3, dans laquelle les petits vésicules unilamellaires contiennent de la phosphatidylcholine et de la phosphatidylsérine.

**5.** Utilisation selon la revendication 4, dans laquelle la phosphatidylcholine et la phosphatidylsérine sont présentes dans un rapport molaire de 1:1.

**6.** Utilisation selon la revendication 3, dans laquelle les petits vésicules unilamellaires sont constitués de phosphatidylcholine et de stéarylamine.

**7.** Utilisation selon l'une quelconque des revendications précédentes, dans laquelle le composé bioactif est le TGF-$\beta$, et les liposomes portent une charge nette négative.

**8.** Utilisation selon l'une quelconque des revendications 1-6, dans laquelle l'agent bioactif est le M-CSF, et les liposomes portent une charge positive nette.

**9.** Agrégats d'un agent bioactif choisi entre le TGF-$\beta$ et le M-CSF, en association avec des liposomes, dans lesquels les agrégats comprennent:
(a) plusieurs liposomes chargés présentant des surfaces extérieures; et
(b) plusieurs agents bioactifs présentant une charge qui est complémentaire de la charge des liposomes;
dans lesquels l'agent bioactif est associé aux surfaces extérieures du liposome, de telle sorte que l'activité biologique de l'agent bioactif ne soit pas essentiellement diminuée lorsque les agrégats sont administrés à un patient en quantité pharmaceutiquement active.

**10.** Agrégats selon la revendication 9, dans lesquels les liposomes sont choisis entre de petits vésicules unilamellaires, de grands vésicules unilamellaires et des vésicules multilamellaires.

**11.** Agrégats selon la revendication 10, dans lesquels les liposomes sont de petits vésicules unilamellaires.

**12.** Agrégats selon la revendication 11, dans lesquels les petits vésicules unilamellaires sont constitués de phosphatidylcholine et de phosphatidylsérine.

**13.** Agrégats selon la revendication 12, dans lesquels la phosphatidylcholine et la phosphatidylsérine sont dans un rapport molaire de 1:1.

18

**14.** Agrégats selon la revendication 11, dans lesquels les petits vésicules unilamellaires sont constitués de phosphatidylcholine et de stéarylamine.

**15.** Agrégats selon l'une quelconque des revendications 9-14, dans lesquels l'agent bioactif est le TGF-$\beta$, et les liposomes sont chargés négativement.

**16.** Agrégats selon l'une quelconque des revendications 9-14, dans lesquels l'agent bioactif est le M-CSF, et les liposomes sont chargés positivement.

**17.** Composition pharmaceutique comprenant:
(a) une quantité pharmaceutiquement active des agrégats selon l'une quelconque des revendications 9-16, et
(b) un support pharmaceutiquement acceptable.

Figure 1

The Effect of Varying Lipid/Protein
Ratios on the Association of TGF-β
With Negatively-Charged Liposomes

EFFECT OF LIPID /PROTEIN RATIO ON ADSORPTION OF TGF−b
ONTO PC/PS LIPOSOMES

Figure 2

The Extent of TGF-β Association
with Neutral Liposomes

PC LIPOSOMES

$^{125}$I—TGFb O—O          $^{14}$C—PC ●—●

Figure 3

The Extent of TGF-β Association
With Negatively-Charged Liposomes

PC/PS LIPOSOMES

$^{125}$I–TGFb  O——O          $^{14}$C–PC  ●——●

# Figure 4

### The Effects of TGF-β Associated Liposomes on Delayed Type Hypersensitivity Responses to <u>Listeria</u>

# Figure 5

## The Effects of TGF-β Associated Liposomes on Delayed Type Hypersensitivity Responses to <u>Listeria</u>

EP 0 393 707 B1